(19) Europäisches Patentamt
European Patent Office
Office européen des brevets

(11) Publication number: 0 307 551 A2

# (12) EUROPEAN PATENT APPLICATION

(21) Application number: 88109710.9

(51) Int. Cl.4: C09D 3/70 , C09D 11/10

(22) Date of filing: 17.06.88

(30) Priority: 22.06.87 US 65250

(43) Date of publication of application: 22.03.89 Bulletin 89/12

(84) Designated Contracting States: CH DE FR GB IT LI NL

(71) Applicant: HENKEL CORPORATION
300 Brookside Avenue
Ambler Pennsylvania 19002(US)

(72) Inventor: Luthy, Chella M.
310 East 46th Street
New York, N.Y. 10017(US)
Inventor: Lovald, Roger A.
1738 Wildwood Lane
Darien Illinois 60559(US)
Inventor: Whyzmuzis, Paul D.
29 W. 251 Wagner Road
Naperville, Illinois 60565(US)
Inventor: Jonaitis, Charles W.
2273 Keim Road
Naperville, Illinois 60565(US)

(74) Representative: von Kreisler, Alek et al
Patentanwälte Von
Kreisler-Schönwald-Fues-Keller
Selting-Werner Deichmannhaus am
Hauptbahnhof
D-5000 Köln 1(DE)

## (54) Fragrance containing polyamide resins useful as coatings and printing inks.

(57) Compositions and methods of printing or coating substrates with aqueous solutions of fragrance containing polymers are disclosed. The fragrance is controllably released from the printing or coating over extended periods of time. The polymers carrying the fragrance are polyamides of fatty acid derived dicarboxylic acids which contain carboxyl groups which will form salts with aqueous solutions of alkaline materials. The alkaline material aids in the solubilization or dispersion of the polyamide in the aqueous solution.

EP 0 307 551 A2

# FRAGRANCE CONTAINING POLYAMIDE RESINS USEFUL AS COATINGS AND PRINTING INKS

## BACKGROUND OF THE INVENTION

### Field of the Invention

This invention relates to the printing or coating from aqueous solutions of fragrance containing polymers on a substrate wherein the fragrance is controllably released over an extended period. The polymers are polyamides of fatty acid derived dicarboxylic acids which contain carboxyl groups which will form salts with aqueous solutions of alkaline materials, the alkaline material being present in a sufficient amount to solubilize or disperse the polyamide in the aqueous solution.

### Background of the Invention

Today, consumers desire a variety of environmental fragrance containing products and many delivery or dispersing systems are employed. These vary from solid or aerosol dispensers to microencapsulated or microsphere containing fragrances which when scratched, or otherwise broken by pressure, release the fragrance to the environment. The former are found in items such as wardrobe sachets, room sprays, fragrance sticks or cones. The latter are found in advertising in magazines or enclosures with the monthly statements. In such materials, a quick release is desired to stimulate the consumer to purchase the article or product containing the fragrance. Such scratch release products are sometimes also found in greeting cards and the like.

In the past, it was found necessary in formulating and using fragrance containing polyamide polymers to employ large amounts of organic solvents such as alcohols or to employ heating devices to release the fragrance to the environment. The release of volatile organic solvents raises environmental concerns. Accordingly, it becomes desirable to apply such fragrance containing polymers from aqueous solutions containing little, if any, organic solvents.

Illustrative of a fragrance releasing composition in which an alcohol is required in U.S. Patent 4,051,159. In this patent there is disclosed compositions of a thermoplastic polyamide resin of polymerized fatty acids and diamines which is used in combination with $C_{14}$-$C_{22}$ alkyl alcohols as the carrier for a fragrance material to provide a fragrance emitting self-supporting article. The alcohol provides for a processing of sufficiently fluid mass at low (about 160 to 170 F.) temperature, thereby retaining the fragrance material during mixing and processing avoiding undue loss of the fragrance material.

Another patent employing polyamide resins of polymerized fatty acids and a perfume along with lower aliphatic alcohols and/or other solvents in U.S. Patent 3,148,125 relating to a lipstick.

U.S. Patent 3,926,655 relates to perfumed polyamide resins. The polyamide resins employed again are those of polymerized fatty acids and diamines such as employed in the other patents noted above. However, in formulating the composition, no co-solvent is necessary for the resin or the perfume oil. The perfume is incorporated by heating to the point where the resin is pourable and stirrable, a hot melt. Relatively rigid polyamide resin bodies were contemplated, without substantial amounts of added co-solvent, which would be useful in objects such as earrings, pins, brooches, decorative castings, etc. Coatings on various substrates are also noted, such as Christmas tree ornaments and electric bulbs such as Christmas tree lights, where heat of the lighted filament increases the rate or release of the perfume oil. The product contains up to 30% by weight of the perfume oil.

U.S. Patent 4,184,099 relates to a composition for slow release of volatile ingredients at high temperatures, again employing polyamide resins of polymerized fatty acids and a diamine. While making reference to application to a substrate from solutions at the top of column 4, and to coated electric light bulbs mentioned in column 6, lines 18-26, the relatively rigid polyamide body containing the perfume is employed in a molded toroidal shape. The perfume is incorporated into the polyamide by heating to a temperature at which the resin is pourable and stirrable, i.e. hot melt. The perfume is incorporated at relatively higher amounts, i.e. 35-70%.

In recent times Alberto-Culver sold decorative products under the trademark "Sparkler", in which a decorative frame such as one resembling a daffodil had voids which were filled with cast films of a

fragrance containing polyamide resin of polymerized fatty acids and a diamine.

Further, pending U.S. application Serial No. 811,619 filed December 20, 1985 by Roger A. Lovald and Leonard R. Vertnik entitled "Polymeric Compositions for Controlled Release of Volatile Materials" (Attorney Docket No. 4303/4306) and assigned to Henkel Corporation relates to certain polyamides of polymerized fatty acids prepared from polyether diamines which are particularly suitable as carriers for fragrances. The composition is molded into articles which can be supported or suspended in an enclosed environment wherein controlled release of the fragrance is desired. The composition is also useful for deposition on the liner of waste receptacles.

While not dealing with fragrance release per se, being filed concurrently with this application is patent application of Roger A. Lovald, Paul D. Whyzmuzis and J.E. Toonen entitled "Aqueous Compositions Containing a Polymeric Fat Acid Polyamide" (Attorney Docket No. 4568), also assigned to Henkel Corporation. This application relates to aqueous compositions containing thermoplastic polymeric fat acid polyamides useful as coatings or adhesives generally.

## SUMMARY OF THE INVENTION

It has now been discovered that certain polyamide resins can be formulated into aqueous solutions or dispersions which are particularly useful in the preparation of fragrance containing coatings. The aqueous solutions containing the fragrance can be applied to a substrate as discontinuous printing or as a coated continuous film which will controllably release the fragrance over an extended period. The polyamide resins which are suitable for use in this invention are certain fatty acid derived polyamide polymers which will form salts in aqueous solutions of alkaline materials which are present in a sufficient amount to solubilize or disperse the polyamide in the aqueous solution. The invention accordingly is concerned with a method of printing or coating on a substrate a fragrance containing fatty acid derived polyamide resins from an aqueous alkaline solution or dispersion thereof.

In general, the method of preparing fragrance containing coatings on a substrate will comprise

(a) forming an aqueous alkaline solution or dispersion of a fragrance containing fatty acid derived polyamide resin wherein the polyamide resin has acid groups which will form salts with the alkaline material present in the aqueous solution whereby the polyamide resin is solubilized;

(b) applying the aqueous alkaline solution containing said polyamide resin to a substrate so as to form a wet film on said substrate;

(c) removing the volatiles from the aqueous wet film applied to the substrate thereby leaving a dry film of said fragrance containing polyamide resin on said substrate.

The volatiles removed will consist essentially of the water, amine or other alkaline materials and/or any alcohol employed in solubilizing or dispersing the polyamide resin as an aqueous dispersion.

The fragrance may be added to the polyamide resin before forming the aqueous dispersion or after forming the aqueous dispersion. As indicated, the film may be discontinuous as when the film is printed on the substrate, or may be a continuous film over the substrate either alone or applied over printing previously applied to the substrate in which the film is accordingly an overlay film.

The polyamides which are suitable for use in the present invention are certain polyamide resins of polymerized fatty acids or acids resulting from the reaction of a fatty acid and an acrylic acid. Illustrative of those derived from the polymerized fatty acids are those prepared from polyether diamine such as described in co-pending application Serial No. 811,619 noted earlier above or in the application being filed concurrently herewith, Attorney Docket No. 4568, noted above. Illustrative of the fatty acid derived products, which are not derived from polymerized fatty acids per se by which are dicarboxylic acids obtained by the Diels-Alder reaction products of acrylic acid and fatty acid having two conjugated ethylenic unsaturated bonds. Such an acid derived from a $C_{18}$ fatty acid is 2-n-hexyl-5-(7-carboxyl-n-heptyl)-cyclohex-3-ene carboxylic acid, a $C_{21}$ acid available from Westvaco, Charleston Heights, South Carolina, as "Westvaco Diacid". Suitable polyamides therefrom, which are substantially free from any polymeric fat acids can also be seen in co-pending U.S. patent application Serial No. 853,090 filed April 17, 1986, by Paul D. Whyzmuzis and John M. Menke (Attorney Docket No. 4360A) entitled "Non-Dimer Polyamides Having Improved Water Solubility Useful as Flexographic/Gravure Ink Binders, assigned to Henkel Corporation, which is a Continuation-in-Part application of Serial No. 701,903 filed February 15, 1985. Other suitable

polyamides. substantially free from any polymeric fat acids, can be seen from co-pending U.S. patent application Serial No. 811.209 (Attorney Docket No. 4517) entitled "Aromatic Dicarboxylic Acid Polyamides", filed December 20, 1985 by Paul D. Whyzmuzis.

## DETAILED DESCRIPTION AND PREFERRED EMBODIMENTS

the method of the present invention in preparing fragrance containing coatings, makes use of certain fatty acid derived polyamide resins, as indicated earlier. The polyamide resins suitable for use herein have an acid value of at least about 10 or greater and are prepared from dibasic, or dicarboxylic acids and difunctional amines, i.e. diamines. The fatty acids employed in the preparation of the polyamides are (a) the polymerized fatty acids which are high in dimeric content, a dicarboxylic functional acid, wherein an unsaturated fatty acid is polymerized to form a dimer containing about twice the number of carbon atoms of the starting acid being polymerized or (b) the difunctional dicarboxylic acid formed by the reaction of a fatty acid with an acrylic acid which provides a dicarboxylic acid in which the number of carbon atoms is the sum of carbon atoms of the fatty acid and the acrylic acid employed. Polyamides of mixtures of (a) and (b) are suitable and the polyamide resins may also be prepared employing additional copolymerizing dicarboxylic acids.

Thus, the polyamide resins suitable for use in the present invention may be generally described as those polyamide resins prepared from a fatty acid dicarboxylic acid and a difunctional amine, wherein the resin has an acid value of about 10, i.e. 8-12 or greater, and which exceeds the amine value.

The polyamides are prepared by reacting the acid components with the diamine components at temperatures in the range of 100 to 300° C. with removal of the water of reaction (or the alcohol, if esters are employed). In general, at the beginning of the reaction it is desirable to use a temperature above 120° C. and preferably above 150° C. to 180° C., with the final temperature of reaction usually above 200° C., preferably about 250° C. The reaction is generally completed in about 1-8 hours dependent on the temperatures employed, with about 2-4 hours at temperatures above 200° C. Substantially equivalent amounts of the acid and amine components are employed, though a relatively small excess of the acidic reactant is employed to provide a product exceeding the amine value and having an acid value on the order of about 10 or higher. As the amount of excess acid is employed, products having higher acid values such as 30 or 50 or higher are obtained.

One of the fatty derived dicarboxylic acids employed are the polymerized fatty acids sometimes referred to as "polymeric fat acids" in which one fatty molecule unites with another fatty molecule to form a dimer dicarboxylic acid. If three molecules react the product is a trimeric tricarboxylic acid.

Polymeric fat acids are well-known and commercially available. One method of preparing polymeric fat acids can be found in U.S. Patent 3,157,681. As used herein the term "polymeric fat acid" refers to a polymerized "fat acid". The term "fat acid" as used herein referes to naturally occurring and synthetic monobasic aliphatic acids having hydrocarbon chains of 8 to 24 carbon atoms. The term "fat acids", therefore, includes saturated, ethylenically unsaturated and acetylenically unsaturated fatty acids. "Polymeric fat radical" is generic to the divalent, trivalent and polyvalent hydrocarbon radicals of dimerized fat acids, trimerized fat acids and higher polymers of fat acids, respectively. These divalent and trivalent radicals are referred to herein as "dimeric fat radical" and "trimeric fat radical", which may be illustrated by the formulae:

$$HOOC-D-COOH$$

and

$$HOOC-\underset{\displaystyle COOH}{\underset{|}{T}}-COOH$$

where D is the divalent hydrocarbon radical of a dimeric dicarboxylic acid and T is the trivalent hydrocarbon radical of a trimeric tricarboxylic acid. In commercially available products, the polymeric fat acid is generally prepared from 18 carbon unsaturated fatty acids such as oleic and linoleic or mixtures thereof providing a dimeric acid containing 36 carbon atoms (D contains 34 carbon atoms) and a trimeric acid containing 54 carbon atoms (T containing 51 carbon atoms).

Because saturated acids are difficult to polymerize and would provide generally low yields of polymeric fat acids, saturated fat acids which include branched and straight acids such as caprylic acid, capric acid, lauric acid, myristic acid, palmitic acid, isopalmitic acid, stearic acid, arachidic acid, behenic acid and lignoceric acid, are not currently commercially significant, such as 10-undecynoic acid, tariric acid, stearolic acid, behenolic acid and isamic acid.

The ethylenically unsaturated acids are much more readily polymerized and are more readily available. Catalytic or non-catalytic polymerization techniques can be employed. The non-catalytic polymerization generally requires a higher temperature. Suitable catalysts for the polymerization include acid or alkaline clays, di-t-butyl peroxide, boron trifluoride and other Lewis acids, antraquinone, sulfur dioxide and the like. Suitable monomers include the branched or straight chain, poly- and mono-ethylenically unsaturated acid such as 3-octenoic acid, 11-dodecenoic acid, linderic acid, lauroleic acid, myristoleic acid, tsuzuic acid, palmitoleic acid, petroselinic acid, oleic acid, elaidic acid, vaccenic acid, gadoleic acid, cetoleic acid, nervonic acid, linoleic acid, linolenic acid, eleostearic acid, hiragonic acid, moroctic acid, timnodimic acid, eicosatetraenoic acid, nisinic acid, scoliodonic acid and chaulmoogric acid.

Because of their ready availability and relative ease of polymerization, oleic and linoleic acids are the preferred starting material for the preparation of the polymeric fat acids. Mixtures of these two acids are found in tall oil fatty acids and, accordingly, commercial tall oil fatty acids are the common source for preparing the polymeric fat acids.

Having obtained the polymeric fat acids or derivatives as described above, they may then be fractionated, for example, by conventional techniques of distillation or solvent extraction. They may be hydrogenated (before or after distillation) to reduce unsaturation under hydrogen pressure in the present of a hydrogenation catalyst.

Typical compositions of polymeric fat acids, based on unsaturated $C_{18}$ tall oil fatty acids, which are the starting materials for the polyamides employed in the present invention, are:

$C_{18}$ monobasic acids ("monomer")0-10% by weight;

$C_{36}$ dibasic acids ("dimer") 70-99.5% by weight;

$C_{54}$ (and higher) ("trimer") polybasic acids 0.2-20% by weight.

The dimer rich fractions (80% and above) are the most desirable, and the preferred starting acids are those containing a dimer acid content of greater than 85% and greater than 90% by weight being the most preferred, which are obtained generally by high vacuum distillation or solvent extraction techniques. The relative ratios of monomer, dimer and trimer (or higher) in unfractionated polymeric fat acids are dependent on the nature of the starting materials and the conditions of polymerization. As is apparent, small amounts of monomeric monocarboxylic acids may be present in the available polymeric fat acids which are, accordingly, a mixture of monomeric, dimeric and trimeric or higher polymeric forms. Accordingly, there is generally present therein some monomeric monocarboxylic acids which contain from 8 to 22 carbon atoms. Other monocarboxylic acids might be present by addition, such as acetic acid, propionic acid and the like. In general, these acids are monomeric, aliphatic hydrocarbon, monocarboxylic acids having from 2 to 22 carbon atoms.

For the purposes of this invention, unless otherwise specified, the "monomeric" (M), "dimeric" (D) and "trimeric" (T) fat acids, were determined by gas liquid chromatography of the corresponding methyl esters. In such method an "intermediate" (I) friction between monomer and dimer is observed. In this method of analysis, the dimeric fat acid content reported is slightly lower than that found by an earlier micromolecular distillation method, which is described in Paschke, R.F., et al., J. Am. Oil. Chem. Soc. XXXI (No. 1), 5 (1954), wherein the distillation is carried out under higher vacuum (below 5 microns) and the monomeric fraction is calculated from the weight of product distilling at 155° C., the dimeric fraction is calculated from that distilling between 155° C. and 250° C., and the trimeric (or higher) fraction is calculated based on the residue.

The other fatty acid derived dicarboxylic acid is one formed by the reaction of a monomeric monocarboxylic fatty acid with an acrylic acid to form a dicarboxylic acid. The $C_{21}$ cycloaliphatic dicarboxylic acid Diels-Alder reaction product of linoleic acid and acrylic acid is described in U.S. Patent 3,753,963, which is a specific fatty acid derived dicarboxylic acid contemplated for use in the present invention. Further, a $C_{21}$ diacid of this type is commercially available as "Westvaco Diacid". 2-n-hexyl-5-(carboxyl-n-heptyl)cyclohex-3-ene carboxylic acid, noted earlier above.

Since the polymeric fat acids are generally prepared from monomeric, monocarboxylic aliphatic acids containing from 16-20, and usually 18 carbon atoms, the dicarboxylic acid therefrom containing twice the number of carbon atoms of the starting monobasic acid are relatively higher carbon atom containing products compared to those prepared by the reaction of the monomeric, monocarboxylic fatty acid with acrylic acid which is a short chain-acid. The polymeric fat acid of a $C_{18}$ monobasic acid such as oleic,

linoleic or linolenic acid will containg about 36 carbon atoms whereas the acid prepared from reaction of the $C_{18}$ fatty acid with acrylic acid will contain 21 carbon atoms.

The difunctional amine compound may be aliphatic, cycloaliphatic, araliphatic and aromatic, including heterocyclic compounds. The compounds may be represented by the formula:

$H_2N - R' - NH_2$

where R' is an aliphatic, cycloaliphatic, araliphatic or aromatic hydrocarbon group containing 2-36 carbon atoms, and may be straight or branched chain. Preferably R' is an alkylene radical having from 2 to 12 carbon atoms. Illustrative of the diamines which may be employed are 1,2-diaminoethane, 1,3,-diaminopropane, 1,4-diaminobutane, 1,6-diaminohexane, 1,10-diaminodecane, 1,12-diaminododecane, 1,18-diaminooctadecane, bis(aminomethyl) benzene, bis(aminoethyl) benzene, cyclohexyl bis(methylamine), isophorone diamine, xylylene diamine, methylene dianiline, and the diamine of a dimeric fat acid, often referred to as dimer diamine. In the dimer diamine prepared from a polymerized $C_{18}$ acid such as oleic or linoleic acid, or mixtures thereof found in tall oil fatty acids, R' will contain 36 carbon atoms. In addition, R' may be branched as in 3,4-diethyl-1,6-diamino-hexane, 3-methyl-1,8-diamino-octane, 2-nonyl-1,10-diaminodecane and 2-octyl-1,11-diaminoundecane.

Diamines in which a hydrocarbon group such as R' are interrupted by oxygen atoms in the chain, which are referred to as ether diamines or polyether diamine may also be employed and can be represented by the general formula

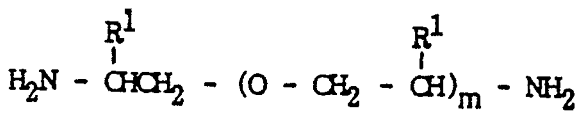

where $R^1$ is hydrogen or a methyl group and m is a positive integer such that the average molecular weight of the polyether diamine is between about 190 and about 3000, more preferably about 400 and 2000. Representative examples of these polyether diamines are available from the Texaco Development Corporation under the trademark "Jeffamine". A particularly useful polyether diamine commercially available is Jeffamine D-2000, wherein R' is a methyl group and m is selected such that the average molecular weight is about 2000, when m is about 33.

Heterocyclic amines such as piperazine or piperidyl compounds may also be employed, and may be defined by the general formula

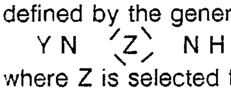

where Z is selected from the group consisting of

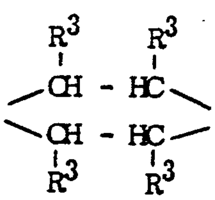

or

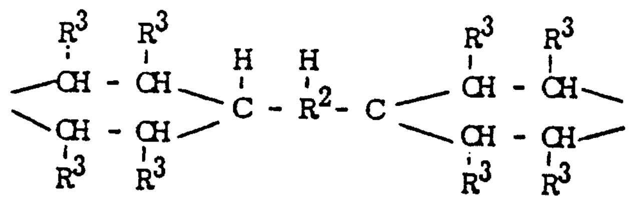

where $R^2$ is a divalent aliphatic hydrocarbon radical having at least 1 carbon atom, generally not exceeding 6 carbon atoms and $R^3$ is hydrogen or an alkyl group having 1-6 carbon atoms, generally methyl, Y is selected from the group of hydrogen and $R^4NH_2$, where $R^4$ is a divalent alkylene group having 1-6 carbon atoms. Suitable available materials corresponding to the formula YNZNH include piperazine, 1,3-di(4-piperidyl) propane, 1,2-di-4-piperidylethane and 1,4-di-4-piperidylbutane.

The amine component of the polyamide may also be an alkanolamine in which case the polyamide also contains ester groups and will accordingly be a polyesteramide. The alkanolamine is difunctional when both the OH groups and $NH_2$ thereof reacting with the carboxyl groups. Accordingly, the equivalents of hydroxyl and amine groups must be considered in the reaction. Such alkanol amines will have the following idealized formula:

$H_2N - Alk - OH$

where Alk is an alkylene group having from 2-6 carbon atoms, preferably 2-3 carbon atoms. Ethanolamine is the preferred alkanolamine employed.

As indicated above, copolymerizing acids other than the fatty acid derived dicarboxylic acids may be employed. Such non-fatty derived copolymerizing acids generally employed are aliphatic, cycloaliphatic or aromatic dicarboxylic acids. These may be ideally defined by the formula:

$R^6OOC - R^5 - COOR^6$

where $R^5$ is an aliphatic, cycloaliphatic, araliphatic or aromatic hydrocarbon radical having from 2 to 20 carbon atoms and $R^6$ is hydrogen or a lower aliphatic hydrocarbon radical having from 2 to 8 carbon atoms such as the alkyl groups, methyl, ethyl, propyl, butyl, hexyl and octyl. The preferred range of these acids is where $R^5$ is a divalent alkylene radical having from 2 to 8 carbon atoms. Illustrative acids are adipic, succinic, glutaric, pimelic, suberic, azelaic, sebacic, terephthalic, isophthalic, cyclohexane dicarboxylic acid, cyclohexane diacetic acid and the like.

The fragrance containing coatings of this invention are prepared by application in the form of an aqueous solution or dispersion of polyamide resin described above either as a continuous or discontinuous film. The fragrance is preferably incorporated into the polyamide after formation of the aqueous solution or dispersion. In incorporating the fragrance prior to formation of the aqueous solution, a low acid value (about 10) polyamide resin (a solid, thermoplastic resin at room temperature) is employed and heated to an elevated temperature above its softening or melting point, generally on the order of about 200-300° F., and the fragrance (not vaporized or volatilized at this temperature, i.e. below flash point of fragrance) added thereto with agitation to provide homogeneity. The resin containing the fragrance may then be cooled and later dissolved in an aqueous alkaline solution, by heating the desired amount of resin, now containing the fragrance, to an elevated temperature about 50° C. and preferably about 70° C., while stirring with agitation until the resin is dispersed. Preferably, the low and high acid value polyamide resins may first be dispersed in the aqueous alkaline solution and the fragrance added to the aqueous dispersion with agitation. The aqueous solution of resin containing the fragrance is then ready for application to a substrate as a wet film, continuous or discontinuous, which after removal of the volatiles leaves a dry film on the substrate which releases the fragrance to the environment over an extended period of time. The volatiles will generally consist of the water from the aqueous dispersion, along with any amine and/or alcohol employed to solubilize the resin.

The suitable fragrances or perfumes include the various natural essential oils, such as lavender oil (which is also useful as an insect repellent) and synthetic analogs of such essential oils which are generally aromatic aldehydes or ketones. Other volatile materials may also be incorporated into the resin such as deodorizer, insect repellents, insecticides, bactericides and animal repellents. The particular volatile materials incorporated will depend largely on the particular environment and purpose desired.

A more exhaustive description of essential oils and perfumes, natural or synthetic, which are suitable for use in the present invention are those described in the Kirk-Othmer Encyclopedia of Chemical Technology, 3rd Edition, Volume 16, John Wiley & Sons, Copyright 1981, pp. 307-332 (Oils, Essential) and pp. 947-971 (Perfumes), the disclosure of which is hereby incorporated by reference.

As indicated earlier, the polyamide resins are dispersed or dissolved in an aqueous alkaline solution. The ability of the composition to be applied from an aqueous solution, having little, if any, organic solvents represents one of the advantages of the present invention.

The alkalinity is achieved with the use of hydroxides, preferably ammonium hydroxide, which leave no metallic hydroxide residue as would the alkali metal or alkaline earth metal hydroxides. Ammonium hydroxide is advantageously employed with small amounts of an alkanol (1-6 carbon atoms), preferably the lower alkanols such as methanol, propanol or ethanol, particularly when employed with polyamide resin prepared from the fatty acids. With resins prepared from the polymerized fatty acids, the alkanolamines, particularly N-alkyl substituted alkanolamine are preferred. The suitable alkanolamines which may be employed are those having the formula

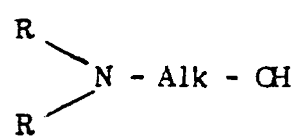

where R is hydrogen or an alkyl group having about 1-6 carbon atoms and each R may be the same or different, and Alk is an alkylene group containing 2-6 carbon atoms. Preferably, the R groups will contain 2-3 carbon atoms such as methyl, ethyl or propyl and the alkylene, Alk, group is an ethylene or propylene group. The N-dialkyl substituted products are preferred, particularly for use with the polyamides prepared

from the polymeric fat acids. The preferred dialkylalkanol amine compounds are dimethylethanolamine and diethylethanolamine.

The alkaline materials should be present in an amount sufficient to solubilize the resin and to form an azeotropic mixture with water. Generally, with the polyamides employed in this invention, the alkaline agent such as the dialkylalkanolamine will be present in the water at a concentration up to about 25% by weight, with about 22-24% being preferred.

Mixtures of the alkaline materials have been found to be particularly useful with the polyamide resins of the polymeric fat acids, such as a mixture of N,N-diethylethanolamine and N,N-dimethylethanolamine, preferably with the diethyl compound predominating as the major component. An especially suitable aqueous solution of dialkylalkanolamine for use with the polymeric fat acid polyamide resin having an acid value of about 10 is a solvent blend composed of about 77% water, 22% N,N-diethylethanolamine and about 1% N,N-dimethylethanolamine. With the polyamide resins of the $C_{21}$ dicarboxylic acid 2-n-hexyl-5-(7-carboxyl-n-heptyl)cyclohex-3-ene carboxylic acid having an acid value on the order of about 90-100, a suitable aqueous solution is one composed by weight of about 75% water, 5% $NH_4OH$ and 20% ethanol.

The invention can best be illustrated by the following examples, in which all parts and percentages are by weight, unless otherwise noted. In the examples to follow, the polyamide resins were prepared by charging the acid and amine reactants to a reactor along with about 1% of an 85% solution of phosphoric acid. The reaction mixture was heated to a temperature within 210-250 °C. and maintained at the temperature for 1 to 2.5 hours, the last 0.5-1 hour being under vacuum. The acid to amine ratio of the reactants employed is adjusted, so as to provide an acid number exceeding the amine number, with an acid number of at least about 10.

## EXAMPLE I

Two polyamide resins were prepared using the typical preparation described. The resins were prepared employing the dicarboxylic acids and difunctional amines indicated.

| Reactant | Resin A Parts | Resin B Parts |
|---|---|---|
| Polymerized Tall Oil Fatty Acids* | 100 | 100 |
| Azelaic Acid | 15.1 | 28.6 |
| Jeffamine® D-2000 | 25.2 | - |
| Piperazine (65% concentration) | 15.9 | 32.4 |
| Ethylene Diamine | 6.8 | 5 |

* Typical analysis 1.5% M (max); 3.5% I (max); 90.0% D (min) and 5.0% T (max)

Both resins A and B had an acid number of about 10 and an amine value of about 1-2.

## EXAMPLE II

Each of resins A and B were dispersed at 20% concentration in an aqueous solvent containing 77.1% by weight water, 21.8% N,N-diethylethanolamine and 1.1% N,N-dimethylethanolamine, by heating the aqueous solvent blend and resin to 70 °C. with stirring until the resin particles could not be seen in the solution.

## EXAMPLE III

In order to illustrate the fragrance carrying ability of the resins and to demonstrate the ability to be formulated into a fragrance printing ink, the aqueous solution of resin A in Example II above was formulated

with a fragrance. This was achieved by adding a fragrance at the level of 20% by weight of the polymer content of solution (1 gram of fragrance to 20 grams of solution or the equivalent of 1 gram of fragrance to 4 grams of polymer).

The solution was then used as an unpigmented printing ink by applying and spreading a thin coat onto a substrate. Coatings were also applied using a rubber printing pad to similate the pad printing process. The coatings resulting from each application were allowed to dry, or were dried by exposure to warm air. This procedure simulates a typical printing process.

After the ink has dried, the added fragrance was retained with the dry resin coating and printing and was observed to last for 200 days after the printing process was complete, thus illustrating the ability of these polyamide coatings or inks to capture the fragrance and release it over extended periods of time. In a similar fashion, resin B will retain fragrance and release it over extended periods of time.

The foregoing example illustrates the capability of the product as an unpigmented varnish for an ink. In formulating the composition as an ink, there would be added to the varnish above pigments and fillers to supply color and opacity. The ink can then be printed on appropriate desired substrates for advertising purposes, greeting cards, packaging materials and the like where a fragrance illustrating the contents as releasing the desired fragrance appropriate to the item on which it is printed or coated. In general, the aqueous based printing ink would be comprised as follows:

Polyamide up to about 40, preferably 10-25% concentration in solvent
Fragrance up to the polyamide weight
Solvent * balance to 100%
Pigment = up to 50% of total ink

Generally, inorganic pigments would be employed in an amount of about 30-50% and organic pigment on the order of 8-20%.

## EXAMPLE IV

Preferred ink formulations employing resin A above are as follows:

| | IV-A parts by weight | IV-B parts by weight |
|---|---|---|
| Inorganic Pigment | 36 | - |
| Organic Pigment | - | 13.00 |
| Resin A | 12.50 | 16.73 |
| Fragrance | 2.50 | 3.35 |
| Dialkylalkanolamine | 11.45 | 15.33 |
| Water | 38.55 | 51.59 |
| | 100.00 | 100.00 |

## EXAMPLE V

Two polyamide resins were prepared employing the $C_{21}$ dicarboxylic acid, 2-n-hexyl-5(7-carboxyl-n-heptyl)cyclohex-3-ene carboxylic acid, using the typical preparation described earlier. The reactants and amounts were as follows:

*water, plus alkaline reagent plus co-solvent alcohol, if any.

| Reactant | Amount (equivalents) | |
|---|---|---|
| | Resin C | Resin D |
| Acid Component: | | |
| $C_2$. Acid | 25 | 35 |
| Adipic Acid | 75 | - |
| Isophthalic Acid | - | 65 |
| Amine Component: | | |
| Isophorone Diamine | 73 | - |
| Hexamethylene Diamine | - | 4.87 |
| Monoethanolamine | - | 79.28 |

Each of the resins had an acid value in the range of 90-100, about 95.

## EXAMPLE VI

A 20% solution of resin D was prepared employing 76 parts water, 4 parts ammonium hydroxide and 20 parts ethanol. A popcorn flavor fragrance was added in an amount of 1 part per 9 parts of polyamide resin varnish solution or 1-8% polyamide resin. The solution was rolled out on paper stock and air drying provided a dry film or coating on the stock which released a popcorn fragrance. A ratio of 2 parts fragrance per 8 parts resin varnish provided a stronger fragrance release. Similar results are obtained when N-propanol is employed in place of the ethanol as the alcohol.

## EXAMPLE VII

An aqueous solution was prepared in which the ratio of fragrance to resin was 50:50 by dissolving the resin (resin C) in an aqueous alkaline solution and adding with mixing the fragrance. The resulting aqueous composition had the following composition:

20 parts resin C
20 parts fragrance (DENEUVE)
12 parts n-propanol
2.56 parts $NH_4OH$
45.44 parts water

The above solution or varnish was printed on paper stock using a flexo hand press. After air drying, the printing released the DENEUVE fragrance (DENEUVE is a Registered Trademark of Avon). Similar results are obtained substituting ethanol for the N-propanol.

In formulating the above varnish with a pigment for printing ink application, inorganic pigments would be employed in an amount of about 35 parts by weight per 65 parts of varnish and organic pigments employed in an amount of about 13 parts with 87 parts of varnish.

## EXAMPLE VIII

Preferred ink formulations emplying resin C are as follows:

| | VIII-A parts by weight | VIII-B parts by weight |
|---|---|---|
| Inorganic Pigment | 35.00 | - |
| Organic Pigment | - | 13.00 |
| Resin C | 13.00 | 17.40 |
| Fragrance | 13.00 | 17.40 |
| n-propanol | 7.80 | 10.44 |
| $NH_4OH$ | 1.66 | 2.23 |
| Water | 29.54 | 39.53 |
| | 100.00 | 100.00 |

As can be seen from the foregoing, the fragrance may be present in an amount up to the amount of resin employed, i.e. 50:50 ratio by weight. Generally, there will be employed ratios by weight of fragrance to resin in the range of about 10:90 to 50:50, more desirably 20:80 to 50:50, with 35:65 or 50:50 being preferred. The amount will largely be dependent on the particular application to which the product will be put and the strength of the fragrance or perfume oil employed. The composition of Example VII has been found to be particularly useful in continuous coatings on ribbons such as decorative ribbons. The composition is also useful (with difference fragrance, however) in spraying and coating of silk artificial flowers to provide a release of the scent appropriate to the silk flower.

The compositions of the resins described are suitable for use in discontinuous film such as in printing on paper or other stock, in letter press, gravure or flexographic ink printing processes. In the examples above, the coatings were air dried, merely allowed to dry at ambient temperatures. In commercial printing or coating operations, heated forced air drying will generally be employed.

## Claims

1. A method of preparing a fragrance carrying coating on a substrate comprising:

(a) forming an aqueous alkaline solution or dispersion of a fragrance containing fatty acid derived polyamide resin wherein said polyamide resin has an acid value of about 10 or higher;

(b) applying said aqueous alkaline solution containing said polyamide resin and fragrance to a substrate so as to form a wet film on said substrate; and

(c) removing volatiles from the aqueous wet film applied to said substrate thereby leaving a dry film of said polyamide resin on said substrate, said polyamide containing said fragrance which is released to the environment.

2. A method as defined in claim 1 wherein said polyamide resin is a polyamide of a polymerized fatty acid.

3. A method as defined in claim 2 wherein said polyamide resin is a polyamide of polymerized tall oil fatty acid.

4. A method as defined in claim 2 wherein said aqueous alkaline solution is comprised of water and an alkanolamine.

5. A method as defined in claim 4 wherein said alkanolamine has the formula

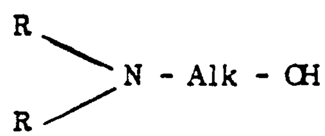

where R is hydrogen or alkyl having 1-6 carbon atoms and wherein the R groups may be the same or different and Alk is an alkylene group having 2-6 carbon atoms.

6. A method as defined in claim 5 wherein said alkanolamine is selected from the group consisting of N,N-diethylethanolamine and N,N-dimethylethanol-amine.

7. A method as defined in claim 4 wherein said aqueous alkaline solution is comprised by weight of 77% water, 22% N,N-diethylethanolamine and about 1% dimethylethanolamine.

8. A method as defined in claim 1 wherein said polyamide resin is a polyamide of a dicarboxylic acid obtained by the reaction of a monocarboxylic fatty acid and an acrylic acid.

9. A method as defined in claim 8 wherein said polyamide resin is a polyamide of 2-n-hexyl-5-(7-carboxyl-n-heptyl)cyclohex-3-ene carboxylic acid.

10. A method as defined in claim 2 wherein said aqueous solution is comprised of water, ammonium hydroxide and an alkanol containing 2-6 carbon atoms.

11. A method as defined in claim 10 wherein said alkanol is selected from the group consisting of ethanol and propanol.

12. A method as defined in claim 8 wherein said aqueous solution is comprised of about 75% water, about 5% ammonium hydroxide and about 20% ethanol.

13. A method as defined in claim 1 wherein said fragrance is incorporated into said polyamide resin prior to forming said aqueous solution.

14. A method as defined in claim 13 wherein said fragrance is incorporated into said polyamide resin by heating said polyamide resin to a temperature above its melt temperature and adding said fragrance to said melt, said fragrance having a flash point above said melt temperature and said polyamide resin having an acid value of about 10.

15. A method as defined in claim 1 wherein said fragrance is incorporated into said polyamide resin after formation of said aqueous solution.

16. A method as defined in claim 15 wherein said fragrance is incorporated into said polyamide resin by adding said fragrance with agitation to said aqueous solution.

17. A method as defined in claim 1 wherein said dry film on said substrate is a substantially continuous film.

18. A method as defined in claim 1 wherein said dry film on said substrate is a discontinuous film.

19. A method as defined in claim 18 wherein said film is printing.

20. A method as defined in claim 19 wherein said substrate is paper stock.

21. A method as defined in claim 17 wherein said substrate is an artificial flower and said fragrance is a flower fragrance.

22. A printing ink composition comprising an aqueous alkaline solution of:

(a) a polyamide resin of a fatty derived dicarboxylic acid wherein said polyamide resin has an acid value of about 10 or higher;

(b) a fragrance capable of being carried by said polyamide resin and of being released to the environment;

(c) an aqueous solvent; and

(d) a pigment

wherein said aqueous solvent is comprised of water containing an alkaline reagent capable of solubilizing said polyamide resin.

23. A printing ink composition as defined in claim 22 wherein said polyamide is present up to a 40% concentration by weight in the aqueous solvent, said fragrance is present in an amount by weight up to the weight of the polyamide present and said pigment is present is an amount of 8-50% by weight of the total ink composition.

24. A printing ink composition as defined in claim 23 in which said pigment if an organic pigment is present in an amount of 8 to about 20% and if an inorganic pigment is present in an amount of about 30 to 50%.

25. A printing ink composition as defined in claim 22 wherein said aqueous solvent is comprised of water an an alkanolamine.

26. A printing ink composition as defined in claim 25 wherein said alkanolamine has the formula:

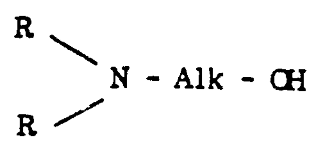

where R is hydrogen or an alkylene group having 1-6 carbon atoms and wherein the R groups may be the same or different and Alk is an alkylene group having 2-6 carbon atoms.

27. A printing ink composition as defined in claim 26 wherein said alkanolamine is selected form the group consisting of N,N- diethylethanolamine and N,N-dimethylethanolamine.

28. A printing ink composition as defined in claim 27 wherein said aqueous solvent is comprised of about 77% water, about 22% N,N-diethylethanolamine and about 1% dimethylethanolamine.

29. A printing ink composition as defined in claim 22 wherein said polyamide is the amidification product of a difunctional amine compound and an acid component comprised of a fatty acid derived dicarboxylic acid compound selected from the group of a polymerized fatty acid and a dicarboxylic acid obtained by the reaction of a monocarboxylic fatty acid and an acrylic acid.

30. A printing ink composition as defined in claim 29 wherein said difunctional amine compound is selected from the group of amine compounds of the for-mulae:

(a) $H_2N - R'NH_2$

(b) 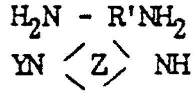

(c) 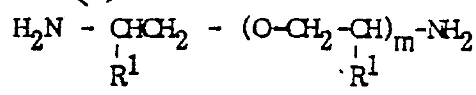

and (d) $H_2N - Alk - OH$

where R′ is an aliphatic, cycloaliphatic, aroliphatic or aromatic hydrocarbon group containing 2-36 carbon atoms; Z is selected from the group consisting of

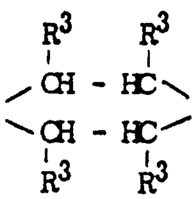

or

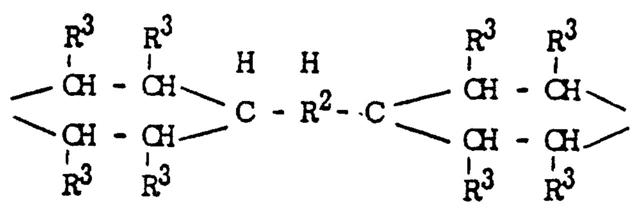

where $R^2$ is a divalent aliphatic hydrocarbon radical having 1-6 carbon atoms, $R^3$ is hydrogen or an alkyl group of 1-6 carbon atoms and Y is selected from the group $R^4NH_2$ where $R^4$ is a divalent alkylene group having 1-6 carbon atoms, $R^1$ is hydrogen or a methyl group and m is a positive integer such that the weight of the polyether diamine (a) is between about 190 to about 3000; and Alk is an alkylene group having from 2-6 carbon atoms.

31. A printing ink composition as defined in claim 30 wherein said acid component further comprises a co-polymerizing dicarboxylic acid of the formula

$R^6 O O C - R^5 - COOR^6$

where $R^5$ is an aliphatic, cycloaliphatic, aroliphatic or aromatic hydrocarbon radical having 2-20 carbon atoms and $R^6$ is H or lower aliphatic hydrocarbon radical having 1-8 carbon atoms.

32. A printing ink composition as defined in claim 29 wherein said polyamide resin is the amidification product of an acid component comprising polymerized tall oil fatty acids and azelaic acid and the amine component is comprised of a mixture of piperazine, ethylene diamine and a polyether diamine of the formula

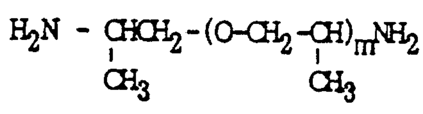

where m is about 33.

33. A printing ink composition as defined in claim 29 wherein said polyamide is the amidification product of an acid component comprising polymerized tall oil fatty acids and azelaic acid and the amine component is comprised of a mixture of piperazine and ethylene diamine.

34. A printing ink composition as defined in claim 29 wherein said polyamid resin is the amidification product of an acid component comprising a mixture of adipic acid and 2-n-hexyl-5(7-carboxyl-n-heptyl)-cyclohex-3-ene carboxylic acid and the amine component is isophorone diamine.

35. A printing ink composition as defined in claim 29 wherein said polyamide resin is the amidification product of an acid component comprising a mixture of isophthalic acid and 2-n-hexyl-5-(7-carboxyl-n-heptyl)cyclohec-3-ene carboxylic acid and the amine component is comprised of a mixture of hexamethylene diamine and monoethanolamine.